# EUROPEAN PATENT SPECIFICATION

(11) **EP 0 264 175 B1**
(45) Date of publication and mention of the grant of the patent: **08.03.1995**
(21) Application number: 87307260.7
(22) Date of filing: 17.08.1987
(51) Int. Cl.: C12N 15/00, C12N 9/99, C07H 21/04, C12N 1/20, C12N 5/00, C12Q 1/68, C12P 21/00

(54) **Protein protease inhibitor from streptomyces**
Proteinartiger Proteaseinhibitor aus Streptomyces
Inhibiteur de protéase à base de protéine de streptomyces

(30) Priority: 18.08.1986 US 897245
(43) Date of publication of application: 20.04.1988
(60) Divisional application: 94110403.6
(73) Proprietor: SMITHKLINE BEECHAM CORPORATION, Philadelphia Pennsylvania 19103 (US)
(72) Inventor: Berka, Thomas Rodney, Wincote Pennsylvania 19095 (US); Fornwald, James Allan, Norristown Pennsylvania 19403 (US); Gorniak, Joselina Gatmaitan, Willow Grove, Pennsylvania 19090 (US); Rosenberg, Martin, Royersford Pennsylvania 19468 (US); Strickler, James Edward, Havertown Pennsylvania 19083 (US); Taylor, Dean Perron, King of Prussia Pennsylvania 19406 (US)
(74) Representative: Valentine, Jill Barbara

(56) References cited:
- EP-A- 0 187 630
- US-A- 4 014 860
- METHODS IN ENZYMOLOGY, vol. 80, 1981, Proteolytic Enzymes, Part C (New York), J. TRAVIS et al., pp. 754-765

## Description

### FIELD OF THE INVENTION

This invention relates to microbial protease inhibitors and, specifically, to a protein protease inhibitors produced by Streptomyces, which is structurally related to Streptomyces Subtilisin Inhibitor, and cloned genes and uses thereof in recombinant DNA processes.

### BACKGROUND OF THE INVENTION

Protein protease inhibitors appear to play a role in regulation of protease functions in living organisms and cells. Such protease inhibitors are widely distributed in animals and plants. Exemplary of these are alpha-1-antiprotease, soybean trypsin inhibitor, bovine pancreatic trypsin inhibitor and antithrombin. Microbially produced protein protease inhibitors include a family of dimeric proteins, each 10 to 12 kilodaltons (kd). This family includes SSI, alkaline protease inhibitor (API-2) from S. griseoincarnatus, plasminostreptin (PSN) from S. antifibrinolyticus and a protease inhibitor from Streptoverticilium cinnamoneum. The inhibitors of the SSI family share extensive sequence homology, e.g., about 70% between SSI and PSN, but appear to have different protease specificities. See, generally, "Protein Protease Inhibitors - The Case of Streptomyces Subtilisin Inhibitor (SSI)", edited by Hiromi et al., Elsevier, 1985, pages 1-14, 139-161 and 365-395. Kakinuma et al., U.S. Patent 4,014,860, disclose PSN and a producing strain thereof.

Protein protease inhibitors have medical application such as in treatment of lung tissue degradation caused by deficiency in alpha-l-antiprotease. Protein protease inhibitors can also be utilized to prevent protein degradation caused by proteases such as are present in serum. Wilson, EP-A-113,319, report use of Erythrina trypsin inhibitor to inhibit conversion of one chain tissue plasminogen activator to the two chain form which occurs in a presence of serum.

Streptomyces are an attractive host for production of desired polypeptides by recombinant DNA techniques because they possess the necessary cellular "machinery" to export proteins and because a great deal of experience in culturing Streptomyces for antibiotic production has been acquired.

A problem which has been encountered in production of heterologous proteins in Streptomyces is protein degradation by endogenous proteases. A second problem which has been encountered is in obtaining export signal sequences which can be fused to heterologous coding sequences to direct export of heterologous gene products.

Furthermore, it is desirable to obtain regulatory regions, e.g., promoters, ribosome binding sites and transcriptional enhancing/stabilizing sequences which can be used to express heterologous coding sequences in Streptomyces at high levels. Such promoters are typically associated with production of abundant mRNA and/or gene products.

Brawner et al., EP-A-187,630 disclose a Streptomyces beta-galacotosidase gene expression unit and use of the promoter and of the export signal sequence thereof to express and export heterologous gene products.

It is an object of the present invention to provide a novel protein protease inhibitor from Streptomyces. It is a further object to provide small exported proteins, which are exported and which are produced in abundant amounts, and DNA coding sequences, export signals and regulatory regions therefor.

### SUMMARY OF THE INVENTION

The invention is of the novel protein protease inhibitor Lividans Exported Protein (10 kd) (LEP-10).

In further aspects of this invention, the invention is a recombinant DNA molecule comprising the LEP-10 gene expression unit, the LEP-10 regulatory region optionally linked to the LEP-10 export signal sequence and/or to a heterologous coding sequence and a microorganism or cell transformed therewith.

### DETAILED DESCRIPTION OF THE INVENTION

LEP-10 is a novel protein protease inhibitor. It is of about the same size as, and shares sequence homology with, the SSI family of protease inhibitors.

LEP-10 was originally identified by Coomassie Brilliant Blue staining of SDS-PAGE protein gels as a low molecular weight (about 10,000 daltons) exported protein present in the medium of a culture of Streptomyces lividans strain 1326. Amino acid sequence data obtained on peptides derived from a tryptic digest of LEP-10 suggested homology with PSN and SSI. Using the amino acid sequence of one of the LEP-10 tryptic peptides, oligonucleotide probes were prepared and used to identify DNA fragments present in S. lividans 1326 chromosomal library which contained a putative LEP-10 sequence. Plasmids containing such putative sequences were used to transform S. albus, which does not naturally produce LEP-10 and transformants were shown to express LEP-10.

A method of cloning and identifying DNA sequences which code for an exported gene product comprises:
1) isolating the gene product from a culture of a producing microorganism or cell and raising specific antisera thereto,
2) cloning fragments of DNA from the producing microorganism or cell into a non-producing strain of Streptomyces,
3) contacting the Streptomyces transformants from step 2 with an adsorbent substrate and incubating the transformants for a time sufficient to allow proteins exported by the transformants to adsorb to the substrate and then
4) assaying the substrate for reactivity with the antisera from step 1.

LEP-10 coding sequence, or sequences for similar proteases, can be identified and isolated from microbial or cell sources by hybridization probing of chromosomal DNA fragments with single-stranded LEP-10 coding sequence fragments. Such probe fragments are preferably of at least about 30 nucleotides in length. A method of identifying DNA coding sequences for protease inhibitors comprises hybridizing fragments of the LEP-10 coding sequences to chromosomal DNA fragments of a microorganism or cell.

The LEP-10 protease inhibitor shares homology with SSI and PSN. LEP-10 is roughly 70% homologous with SSI and PSN. Following is the amino acid sequence of mature LEP-10 as determined by amino acid sequencing of tryptic digests and/or DNA sequence analyses. Amino acid analyses were carried out in a Beckman 890M amino acid sequenator (Beckman Instruments, Fullerton, California). The sequences of SSI and PSN from published reports are shown where they differ from LEP-10.

10
LEP-10 NH2-SER-LEU-TYR-ALA-PRO-SER-ALA-LEU-VAL-LEU-THR-VAL-
PSN NH2-GLY- -MET-
SSI ASP-ALA-PRO-SER-ALA-

20
LEP-10 GLY-HIS-GLY-GLU-SER-ALA-ALA-THR-ALA-ALA-PRO-LEU-ARG-ALA-VAL-
PSN -ASN- -THR-VAL-ASN- -GLU-
SSI -LYS- -VAL- -THR-THR- -GLU-

30 40
LEP-10 THR-LEU-THR-CYS-ALA-PRO-THR-ALA-SER-GLY-THR-HIS-PRO-ALA-ALA-
PSN -ASN-
SSI -GLY-PRO-

50
LEP-10 ALA-ALA-ALA-CYS-ALA-GLU-LEU-ARG-GLY-ALA-HIS-GLY-ASP-PRO-SER-
PSN LEU-GLN- -GLY- -PHE-ASP-
SSI GLY-SER- -ASP- -ALA-ALA-VAL-GLY- -LEU-ASN-

60 70
LEP-10 ALA-LEU-ALA-ALA-GLU-ASP-SER-VAL-MET-CYS-THR-ARG-GLU-TYR-ALA-
PSN -THR-VAL-ARG-GLY-ASP- -ALA- -LYS-GLN-PHE-ASP-
SSI -THR-ARG-GLY-GLU-ASP- -PRO-MET-VAL- -ASP-

80
LEP-10 PRO-VAL-VAL-VAL-THR-VAL-ASP-GLY-VAL-TRP-GLN-GLY-ARG-ARG-LEU-
PSN -LYS-ARG-VAL-
SSI -LYS-ARG-VAL-

90 100
LEP-10 SER-TYR-GLU-ARG-THR-PHE-ALA-ASN-GLU-CYS-VAL-LYS-ASN-ALA-GLY-
PSN -THR- -SER-TYR-
SSI -VAL- -SER- -GLU-MET- -HIS-

LEP-10 SER-ALA-SER-VAL-PHE-THR-PHE-COOH
PSN GLY-MET-THR- -PHE-COOH
SSI GLY-SER- -ALA-PHE-COOH

The above sequence is substantially accurate and, in any event, is not limiting of the invention. It is understood that other LEP-10 sequences may be obtained due, for example, to variations which do not significantly affect activity, alternative processing or analytical error.

LEP-10 has been shown to inhibit trypsin in a standard trypsin inhibition assay. See, Travis, Meth. Enzym., 80:755-765 (1981). Thus, LEP-10 can be used in medical and other applications in which trypsin inhibition, or other protease inhibition is desired.

Trypsin inhibitory activity was determined in a chromogenic assay using KABI S2288 as substrate (HO-ILE-PRO-ARG-p-nitroanilide.) Scisson of the ARG-p-nitroanilide bond releases p-nitroaniline which absorbs at 410 nm; thus, the extent of cleavage is measured by the increase in absorbance at 410 nm. This assay was used in qualitative manner to determine the inhibitory activity of LEP-10. Briefly, serial two fold dilutions of either conditioned medium (CM) or ammonium sulfate concentrates (AS) of conditioned medium were mixed with 25 ng of trypsin and the substrate was added. After 30 min at room temperature, the assay was stopped by addition of acetic acid. The assay was carried out in microtiter plates, which were then read in an ELISA reader. Similar samples of PSN were included as controls. The data shown are the absorbances measured following cleavage of the chromogenic substrate by trypsin. The growth medium itself shows no activity (data not shown).

| Reciprocal Dilution | Inhibitor | | |
|---|---|---|---|
| | PSN CM | PSN AS | LEP-10 AS |
| No. Inh. | 0.772 | 0.751 | n.d. |
| 1 | 0.010 | 0.018 | 0.070 |
| 2 | 0.046 | 0.021 | 0.091 |
| 4 | 0.046 | 0.023 | 0.130 |
| 8 | 0.053 | 0.024 | 0.165 |
| 16 | 0.058 | 0.036 | 0.219 |
| 32 | 0.273 | 0.076 | 0.293 |

LEP-10 showed high activity compared to PSN. However, because the dilutions used were all too low to reach a 50% inhibition level the activities cannot be closely compared accurately. ("n.d." means not detected.)

The protein can also be used to inhibit endogenous Streptomyces or other proteases and thereby to inhibit degradation of desirable proteinaceous products, including heterologous proteins produced by recombinant DNA techniques. For this purpose, the inhibitor can be added to a Streptomyces or other, e.g., E. coli, Bacillus, yeast, insect or mammalian, cell culture. Alternatively, Streptomyces strains or other hosts can be genetically engineered to produce the inhibitor along with a protein of interest, as described further below.

The protein can also be used to inhibit degradation of proteins in protease-containing solutions and as laboratory reagents such as in an assay for protease inhibition.

The gene expression unit for LEP-10, that is, the DNA sequence containing the LEP-10 coding sequence and regulatory regions required for transcription and translation, was localized on a 4 kb PstI fragment of S. lividans 1326 chromosomal DNA and, furthermore, within a 2.97 kb BamHI-PstI fragment thereof.

The LEP-10 gene expression unit can be further isolated by additional restriction endonuclease or endonuclease/exonuclease digests and cloning and expression of fragments thereof or by further DNA sequencing. By similar techniques, the coding sequence alone can be isolated and cloned, such as in an expression vector and the regulatory region alone can be isolated and cloned, such as in a promoter probe vector. More specifically, the coding sequence can be fused in-frame to a promoter in a plasmid. Such plasmid is used to transform a Streptomyces or other, e.g., E. coli, B. subtills, yeast, insect or mammalian host. Such recombinant host is then cultured and the medium or cell extracts are screened for presence of the inhibitor. Screening can be, for example, by gel electrophoresis of proteins, wherein the inhibitor can be detected as a 10 kd protein, by a trypsin inhibition assay, by immunodetection using anti-LEP-10 antibody, by hybridization with LEP-10 probes or fragments, and/or by amino acid composition analysis or sequencing of putative LEP-10 proteins. Exemplary of promoters known to function in Streptomyces are the Streptomyces beta-galactosidase promoter, the leftward promoter of lambda (PL), the tyrosinase promoter and promoters of genes conferring antibiotic resistance such as erythromycin resistance, neomycin resistance, and thiostrepton resistance.

The regulatory region of LEP-10 can be inserted into a promoter probe vector, that is, one having a coding sequence for a readily detectable phenotypic marker such that following insertion of a functional promoter upstream of the marker sequence the sequence is expressed. Exemplary of markers useful in Streptomyces are: antibiotic resistance markers, e.g., thiostrepton, kanamycin and the Streptomyces β-galactosidase.

The LEP-10 regulatory regions can be used to express LEP-10 in the native gene expression unit or to express heterologous polypeptides or proteins in a hybrid gene expression unit as transcriptional or translational fusions. For example, the promoter of LEP-10 can be fused in frame upstream of a coding sequence for vaccine antigens, e.g., hepatitis B surface antigen and rabies glycoprotein; or for pharmacologically active proteins, e.g., interleukins, plasminogen activators, other protease inhibitors such as alpha-l-antiprotease, tumor necrosis factor, Factor VIII and influenza NSl. The export signal of LEP-10 can likewise be isolated and ligated to a coding sequence for a polypeptide which is not normally excreted, in frame and downstream of a promoter.

Functional derivatives of each domain within the gene expression units of the invention, i.e., promoter functions, export functions, and protease inhibition functions, can be prepared by use of restriction endonucleases, random mutagenesis such as by ultraviolet irradiation and site directed mutagenesis such as by insertion, addition or substitutions of synthetic oligonucleotides. Such derivatives can readily be checked for effect on function. See, e.g., Davis et al., "Adv. Bact. Genetics", Cold Spring Harbor Laboratory (1980); Miller, "Experiments in Molecular Genetics", Unit III, Cold spring Harbor Laboratory (1972); Botstein et al., Science 229:1193 (1985); and Estell et al., Science 233:659 (1986). Functional derivatives of the coding sequences, and of variant proteins produced thereby, are included within the scope of this invention.

Functional derivatives of the proteins can also be prepared by directly altering the proteins. This can be accomplished by chemical means including cleavage to remove amino acids and insertion or addition of amino acids. Such chemically prepared derivatives can be checked for activity such as by a trypsin inhibition assay. Functional chemically prepared derivatives are also included in the scope of the invention.

Following is the DNA sequence of the LEP-10 coding sequence and for some upstream and downstream sequences. The signal cleavage site is indicated. The following sequence is substantially accurate and is not limiting of the invention. For example, the illustrated sequence may be incorrect in one or a few base pairs and/or amino acids. Also, other sequences which also code for LEP-10 may exist or may be fabricated, which other sequences may differ in one or a few base pairs and/or amino acids. Such other sequences are expected to be at least about 90% homologous with the illustrated DNA sequence.

-390 -380 -370 -360 -350 -340
* * * * * *
GCA CGG GGA TGT AGG GGA GTC GGG CGG AGG CCG CGA CCG AGG CCA GGG CGC GTC CCG

-330 -320 -310 -300 -290 -280
* * * * * *
GCG TGC ACC GAC AGC AGG TTG TCC ATC CGC GCC GCC ACC AGC TCC CGG TCG CGG CCC

-270 -260 -250 -240 -230
* * * * *
AGG TAG CCG GGG GCT CCA CGG AGT GCG TCA TCA GGT CCC AGC CGG TGA ACC TCG CCG

-220 -210 -200 -190 -180 -170
* * * * * *
GCC AGC CCC [?]CC TCG TCC TCC AGG AAC GCT AAT CAA GAA ACG CCC AGT TCC GGA CTT

-160 -150 -140 -130 -120 -110
* * * * * *
GGA ACG TTC TAA TTC TGT GAC TTC ACG CCA CTG ATT CAA TAC GCA AGG TTA CCG AAC

-100 -90 -80 -70 -60
* * * * *
ACC GTG GGG TCG AGA TGA GTT TGC GTG CCG GGA CTC GGC AGA CTC GCC GCT CCG GCA

-50 -40 -30 -20 -10 1
* * * * * *
CCG ACC GGG TGC ACC GGC ACC ACC CTC GAA ACG AGC GGA AGG ATG CAC ACA ATG CGG
Met Arg

10                  20                  30                  40                  50                  60
*                   *                   *                   *                   *                   *
AAC ACC GCG CGC TGG GCA GCG ACT CTG GGC CTG ACG GCC ACC GCC GTC TGC GGG CCC
Asn Thr Ala Arg Trp Ala Ala Thr Leu Gly Leu Thr Ala Thr Ala Val Cys Gly Pro

70                  80                  90                  100     SIGNAL CLEAVAGE 110                 120
*                   *                   *                   *                   *                   *
CTC GCC GGG GCC TCC CTC GCC TCC CCG GCC ACC GCC CCC GCG | TCG CTC TAC GCC CCC
Leu Ala Gly Ala Ser Leu Ala Ser Pro Ala Thr Ala Pro Ala | Ser Leu Tyr Ala Pro

130                 140                 150                 160                 170
*                   *                   *                   *                   *
TCG GCC CTG GTG CTG ACC GTG GGG CAC GGA GAG AGC GCT GCC ACC GCC GCA CCC CTG
Ser Ala Leu Val Leu Thr Val Gly His Gly Glu Ser Ala Ala Thr Ala Ala Pro Leu

180                 190                 200                 210                 220                 230
*                   *                   *                   *                   *                   *
CGC GCG GTC ACC CTG ACC TGC GCC CCG ACC GCC TCC GGC ACC CAC CCG GCG GCC GCC
Arg Ala Val Thr Leu Thr Cys Ala Pro Thr Ala Ser Gly Thr His Pro Ala Ala Ala

240                 250                 260                 270                 280                 290
*                   *                   *                   *                   *                   *
GCG GCC TGT GCC GAA CTG CGC GCC GCG CAC GGC GAC CCG AGT GCC CTG GCC GCC GAG
Ala Ala Cys Ala Glu Leu Arg Ala Ala His Gly Asp Pro Ser Ala Leu Ala Ala Glu

300                 310                 320                 330                 340
*                   *                   *                   *                   *
GAC TCG GTG ATG TGC ACC CGG GAG TAC GCC CCC GTG GTC GTC ACC GTC GAC GGC GTC
Asp Ser Val Met Cys Thr Arg Glu Tyr Ala Pro Val Val Val Thr Val Asp Gly Val

350                 360                 370                 380                 390                 400
*                   *                   *                   *                   *                   *
TGG CAG GGG CGG CGC CTC TCC TAC GAA CGC ACC TTC GCC AAC GAG TGC GTG AAG AAC
Trp Gln Gly Arg Arg Leu Ser Tyr Glu Arg Thr Phe Ala Asn Glu Cys Val Lys Asn

410                 420                 430                 440                 450                 460
*                   *                   *                   *                   *                   *
GCG GGC AGC GCG AGC GTC TTC ACG TTC TGA GGG ACC GGG ACC GCC GGA CTG CGC GTG
Ala Gly Ser Ala Ser Val Phe Thr Phe ---

470                 480                 490                 500                 510
*                   *                   *                   *                   *
ATC GGC TGC TCG CTA CTG GGG AGT GCG AGC GCC GCC GTA CGG GTC CCG CGG GCC CGC

520                 530                 540                 550                 560                 570
*                   *                   *                   *                   *                   *
ACC GGG GAC GGC GGA CGG AGT GGG GCC GTC CGC CGT TCT CCC GGT TCA GGG GCA CGG

580                 590                 600                 610                 620                 630
*                   *                   *                   *                   *                   *
TCG GCC GTT CGC GGC CGG GCC GTC CGC GGC CCT GGC GGA CCG GGC ACG GTG GCT GTG

640                 650
*                   *
TCG CAC CAC GGA TAG CGC CGA CTG

The LEP-10 protein, as noted above, was originally discovered as a product of S. lividans 1326 and S. longisporus. It is likely, however, that other strains and species produce the same or substantially the same proteins, i.e., substantially homologous (>90%, especially >95%) and substantially the same substrate (protease) specificity and activity. Also, the LEP-10 protein can be synthesized by standard protein synthesis techniques or DNA coding sequences therefor can be synthesized by standard DNA synthesis techniques. Thus, the invention includes the LEP-10 protein and gene expression unit and functional domains thereof regardless of source.

Oligonucleotide probes for detecting similar DNA sequences in other strains and species can be synthesized based on the LEP-10 sequences. However, as noted above, results of probing with such oligonucleotides may produce false positives. Therefore, probing initially or secondarily with larger DNA fragments, e.g., greater than about 30 nucleotides, and preferably greater than about 50 nucleotides, is preferred. By such probing techniques, genes encoding the same inbibitors or other inhibitors of the SSI family can be identified.

By recombinant DNA techniques, the coding sequences of the invention can be used to produce large quantities of LEP-10. These techniques comprise, in sum, transformation of a host, bacterial or eukaryotic, with a gene expression unit of the invention comprising the LEP-10 coding sequence. For this purpose, the native gene expression unit on a plasmid or other vector or a hybrid gene expression unit comprising the inhibitor coding sequence and a heterologous regulatory region can he used. The inhibitors so produced are purified to a desirable extent by standard protein isolation techniques.

The following Examples are illustrative, and not limiting of the invention.

### EXAMPLES

### Example 1. LEP-10

S. lividans strain 1326 (Agricultural Research Culture Collection, Peoria, Illinois, NRRL 15091) was cultured in SL-glycerol, SL-glucose and YEMES broths at 28°C for approximately 30 hours. After spinning down the cells by low speed centrifugation, the supernatants were concentrated by ammonium sulfate precipitation, redissolved and electrophoresed on SDS-PAGE (15%) gels to separate protein products. Upon staining with Coomassie Brilliant Blue R-250, a dense band corresponding to a protein having a molecular weight of about 10,000 daltons was identified as having been expressed in certain broths. SL medium comprises components SL-A and SL-B and a trace elements solution as follows:

| SL-A | |
|---|---|
| (NH₄)₂SO₄ | 1.0 g/l |
| L-asparagine | 2.0 g/l |
| K₂HPO₄ | 9.0 g/l |
| NaH₂PO₄ | 1.0 g/l |

| SL-B | |
|---|---|
| yeast extract | 20 g/l |
| MgCl₂ê6H₂O | 5.0 g/l |
| CaCl₂êH₂O | 0.1 g/l |
| trace elements sol. | 20 ml/l |

| Trace elements sol. | |
|---|---|
| ZnCl₂ | 40 mg/l |
| FeCl₃ê2H₂O | 200 mg/l |
| CuCl₂ê2H₂O | 10 mg/l |
| MnCl₂ê4H₂O | 10 mg/l |
| Na₂B₄O₇ê10H₂o | 10 mg/l |
| (NH₄)₆Mo₇O₂₄ê4H₂O | 10 mg/l |

SL-A is autoclaved and SL-B is filter sterilized prior to adding together in a ratio of 1:10 SL-B: SL-A (v/v). To prepare SL-glucose and SL-glycerol, 1% (w/v) glucose and glycerol, respectively, are added.

Samples of the protein were cut out and removed from gels. The protein was reduced and S-dansylamidoethylated with dansylazridine. The alkylated protein was digested with trypsin and the tryptic peptides were recovered by reverse phase HPLC. Individual peptides were sequenced in a Beckman sequenator to obtain the primary structure data reported above.

Based on the amino acid sequence, a series of single stranded oligonucleotide probes were prepared. Probe #263 was a mixed 24-mer as follows: 
G
3'CGGAAGCGCTTGCTCACGCAGTTC5'
C T T C
These probes were used to probe a Charon phage library of S. lividans 1326 chromosomal DNA prepared substantially as described by Maniatis et al., "Molecular Cloning-A Laboratory Manual," 1982, Cold Spring Harbor Laboratory.

The recombinant phage, Charon 25.5 (Ch25.5), contains Streptomyces lividans 1326 chromosomal DNA which hybridizes to the mixed oligonucleotide probe #263. This probe is complementary to the LEP-10 mRNA. A BglII-EcoRI fragment (about 18 kb) of Ch 25.5 was cloned into the Bam HI-EcoRI site of plasmid pUC18 to yield plasmid pDl.

From pDl was isolated a PstI fragment (about 4 kb) which also hybridized with the oligonucleotide probe. This PstI fragment was cloned into the PstI site of pBR322 to yield plasmid pBR33. The 4 kb PstI fragment contains a BamHI-PstI (about 2.97 kb) fragment which hybridizes with oligonucleotide probe.

Both the 4 kb PstI and the 2.97 kb Bam HI-PstI fragments were cloned into the shuttle plasmid vector pMB157, which is a low copy number E. coli-Streptomyces shuttle vector comprising SCP2 stability and replication functions, pUC8 sequences and thiostrepton and ampicillin resistance. The recombinant plasmids pMB157-21 and pMB157-8 (which contain the 2.97 kb BamHI- PstI fragment) and pMB157-22 (which contains the 4 kb PstI fragment) were transformed into protoplasts of S. albus which was previously shown not to produce LEP-10. Colonies of transformants were checked for the production of LEP-10 protein by immunoblotting. Nitrocellulose filters (0.2 um) were applied directly to plates containing colonies of the transformants. After the filters were removed from the plates they were processed with antibody to LEP-10 by the Western blot procedure. Transformants of S. albus containing the recombinant plasmids produced extracellular LEP-10 as determined by this immunoblot procedure. LEP-10 in culture supernatants of S. albus (pMB157-22) and S. albus (pMB157-21) grown in SL-glycerol medium was also detected by Western blots of SDS-PAGE gels. Thus, all of the information necessary to produce a mature extracellular LEP-10 protein in S. albus is present on the 2.97 kb Bam HI-PstI fragment.

Partial sequence of the LEP-10 gene has been identified on an RsaI fragment (about 180 bp) isolated from the recombinant phage Ch25.5. This RsaI fragment is present in both the aforementioned 4 kb PstI and the 2.97 kb BamHI-PstI fragments. The RsaI fragment contains a sequence encoding the carboxy-terminal one-third of the LEP-10 protein.

### Example 2. Heterologous gene expression and secretion using LEP-10 regulatory regions

A LEP-10-IL-lB fusion was constructed in pUC 18. The fusion contained S. lividans DNA from a HinfI site (between bases -132 and -133 in the above-illustrated sequence for LEP-10) to a cut-back BglI site (between bases 85 and 86). The blunt-ended Bg1I site was ligated to a filled-in AvaI site in pUC18. Then, a DNA fragment containing the BanI IL-1B fragment with a BamH1 linker was ligated to a BamH1 site in puC 18 to yield the construction illustrated as follows: 
1                2              3              4
|***CTC GCC T|CC GGG| GAT CCG| GCA CCT***|
wherein region 1 is derived from LEP-10, region 2 is derived from pUC 18, region 3 is from the linker and region 4 is the lL-lB coding sequence. Maintenance sequences from the Streptomyces vector, pIJ102 (Kieser et al., Mol. Gen. Genet. 185:223 (1982)), were then inserted.

Following transformation of S. lividans 12K and culturing transformants in trypticase soy broth, an extracellular protein which corresponds approximately to mature IL-lB was observed by western immunoblotting. Although N-terminal sequence analysis of the excreted protein has not been carried out, it appears that cleavage may occur between the first residue (alanine) and the second residue (proline) of IL-lB to remove the LEP-10 signal sequence.

Example 2 demonstrates utility of the LEP-10 regulatory region both to express heterologous gene products in Streptomyces and to export heterologous gene products which are not otherwise

The above description and examples fully describe the invention and preferred embodiments thereof. The invention, however, is not limited to precisely the embodiments described but also include all modifications coming within the scope of the claims which follow.

## Claims (Claims for the following Contracting State(s): BE, CH, DE, FR, GB, IT, LU, NL, SE, GR)

1. A recombinant DNA molecule comprising the LEP-10 regulatory region located on the HinfI - BglI subfragment of the 2.97kb BamHI - PstI fragment of a 4kb PstI fragment of S. lividans 1326 chromosomal DNA.

2. The DNA molecule of claim 1 wherein said region comprises the sequence:

-130 -120 -110
ATT CAA TAC GCA AGG TTA CCG AAC

-100 -90 -80 -70 -60
ACC GTG GGG TCG AGA TGA GTT TGC GTG CCG GGA CTC GGC AGA CTC GCC GCT CCG GCA

-50 -40 -30 -20 -10 1
CCG ACC GGG TGC ACC GGC ACC ACC CTC GAA ACG AGC GGA AGG ATG CAC ACA A

3. The DNA molecule of claim 1 or 2 wherein the regulatory region is operatively linked to the LEP-10 export signal sequence located within said subfragment.

4. The DNA molecule of claim 1, 2 or 3 wherein the regulatory region is operatively linked to a heterologous coding sequence.

5. A recombinant DNA molecule comprising the LEP-10 gene expression unit located within a 2.97kb BamHI-PstI fragment of a 4kb PstI fragment of S.lividans 1326 chromosomal DNA.

6. An isolated DNA molecule having the sequence 
-390 -380 -370 -360 -350 -340
* * * * * *
GCA CGG GGA TGT AGG GGA GTC GGG CGG AGG CCG CGA CCG AGG CCA GGG CGC GTC CCG

-330 -320 -310 -300 -290 -280
* * * * * *
GCG TGC ACC GAC AGC AGG TTG TCC ATC CGC GCC GCC ACC AGC TCC CGG TCG CGG CCC

-270 -260 -250 -240 -230
* * * * *
AGG TAG CCG GGG GCT CCA CGG AGT GCG TCA TCA GGT CCC AGC CGG TGA ACC TCG CCG

-220 -210 -200 -190 -180 -170
* * * * * *
GCC AGC CCC [?]CC TCG TCC TCC AGG AAC GCT AAT CAA GAA ACG CCC AGT TCC GGA CTT

-160 -150 -140 -130 -120 -110
* * * * * *
GGA ACG TTC TAA TTC TGT GAC TTC ACG CCA CTG ATT CAA TAC GCA AGG TTA CCG AAC

-100 -90 -80 -70 -60
* * * * *
ACC GTG GGG TCG AGA TGA GTT TGC GTG CCG GGA CTC GGC AGA CTC GCC GCT CCG GCA
 
-50 -40 -30 -20 -10 1
CCG ACC GGG TGC ACC GGC ACC ACC CTC GAA ACG AGC GGA AGG ATG CAC ACA ATG CGG
Met Arg

10 20 30 40 50 60
AAC ACC GCG CGC TGG GCA GCG ACT CTG GGC CTG ACG GCC ACC GCC GTC TGC GGG CCC
Asn Thr Ala Arg Trp Ala Ala Thr Leu Gly Leu Thr Ala Thr Ala Val Cys Gly Pro

70 80 90 100 SIGNAL CLEAVAGE 110 120
CTC GCC GGG GCC TCC CTC GCC TCC CCG GCC ACC GCC CCC GCG TCG CTC TAC GCC CCC
Leu Ala Gly Ala Ser Leu Ala Ser Pro Ala Thr Ala Pro Ala Ser Leu Tyr Ala Pro

130 140 150 160 170
TCG GCC CTG GTG CTG ACC GTG GGG CAC GGA GAG AGC GCT GCC ACC GCC GCA CCC CTG
Ser Ala Leu Val Leu Thr Val Gly His Gly Glu Ser Ala Ala Thr Ala Ala Pro Leu

180 190 200 210 220 230
CGC GCG GTC ACC CTG ACC TGC GCC CCG ACC GCC TCC GGC ACC CAC CCG GCG GCC GCC
Arg Ala Val Thr Leu Thr Cys Ala Pro Thr Ala Ser Gly Thr His Pro Ala Ala Ala

240 250 260 270 280 290
GCG GCC TGT GCC GAA CTG CGC GCC GCG CAC GGC GAC CCG AGT GCC CTG GCC GCC GAG
Ala Ala Cys Ala Glu Leu Arg Ala Ala His Gly Asp Pro Ser Ala Leu Ala Ala Glu

300 310 320 330 340
GAC TCG GTG ATG TGC ACC CGG GAG TAC GCC CCC GTG GTC GTC ACC GTC GAC GGC GTC
Asp Ser Val Met Cys Thr Arg Glu Tyr Ala Pro Val Val Val Thr Val Asp Gly Val

350 360 370 380 390 400
TGG CAG GGG CGG CGC CTC TCC TAC GAA CGC ACC TTC GCC AAC GAG TGC GTG AAG AAC
Trp Gln Gly Arg Arg Leu Ser Tyr Glu Arg Thr Phe Ala Asn Glu Cys Val Lys Asn

410 420 430 440 450 460
GCG GGC AGC GCG AGC GTC TTC ACG TTC TGA GGG ACC GGG ACC GCC GGA CTG CGC GTG
Ala Gly Ser Ala Ser Val Phe Thr Phe ---

470 480 490 500 510
ATC GGC TGC TCG CTA CTG GGG AGT GCG AGC GCC GCC GTA CGG GTC CCG CGG GCC CGC

520 530 540 550 560 570
ACC GGG GAC GGC GGA CGG AGT GGG GCC GTC CGC CGT TCT CCC GGT TCA GGG GCA CGG

580 590 600 610 620 630
TCG GCC GTT CGC GGC CGG GCC GTC CGC GGC CCT GGC GGA CCG GGC ACG GTG GCT GTG

640 650
TCG CAC CAC GGA TAG CGC CGA CTG
 which includes the LEP-10 encoding region.

7. A vector comprising the DNA molecule of any of claims 1 to 6.

8. A host microorganism or cell transformed with the DNA molecule of any of claims 1 to 6, or the vector of claim 7.

9. An isolated DNA molecule consisting essentially of the LEP-10 regulatory/export region HinfI - BglI subfragment of the 2.97kb BamHI - PstI fragment of a 4kb PstI fragment of S. lividans 1326 chromosomal DNA.

10. A process for preparing a DNA molecule comprising the LEP-10 regulatory region as claimed in claim 1 or 9 which process comprises hybridizing the chromosomal DNA to a probe based on the LEP-10 regulatory region to identify chromosomal DNA regions which comprise the LEP-10 regulatory region and then isolating the identified chromosomal DNA regions by restriction with a restriction endonuclease.

11. A process for preparing a DNA molecule comprising the LEP-10 regulatory region operatively linked to the LEP-10 export signal sequence as claimed in claim 3 or 9 which process comprises hybridizing the chromosomal DNA to a probe based on the LEP-10 regulatory region and export signal sequence to identify chromosomal DNA regions which comprise the LEP-10 regulatory region and export signal sequence and then isolating the identified chromosomal DNA regions by restriction with a restriction endonuclease.

12. A process for preparing a recombinant DNA molecule as claimed in claim 4 which process comprises restricting a recombinant DNA molecule having all or a portion of the LEP-10 gene expression unit at a point downstream of the LEP-10 regulatory region and ligating to said portion a coding sequence for a heterologous peptide.

13. A process for preparing a recombinant DNA molecule comprising the LEP-10 gene expression unit as claimed in claim 5 or 6 which process comprises hybridizing chromosomal DNA to a probe based on the LEP-10 gene expression unit to identify chromosomal DNA regions which comprises the LEP10 gene expression unit and then isolating the identified chromosomal DNA regions by restriction with a restriction endonuclease.

14. A process for producing a polypeptide in Streptomyces which comprises transforming Streptomyces organisms with the recombinant DNA molecule of claim 4, 5 or 6 and then culturing the transformed Streptomyces such that the polypeptide is expressed.

15. A process for preparing a DNA molecule as defined in any one of claims 1 to 6 or 9 or vector as defined in claim 7 which process comprises fusing appropriate DNA fragments.

## Claims (Claims for the following Contracting State(s): AT, ES)

1. A process for preparing a recombinant DNA molecule comprising the LEP-10 regulatory region located on the HinfI - BglI subfragment of the 2.97kb BamHI - PstI fragment of a 4kb PstI fragment of S. lividans 1326 chromosomal DNA, which process comprises fusing appropriate DNA fragments.

2. A process according to claim 1 wherein said region comprises the sequence:

-130 -120 -110
ATT CAA TAC GCA AGG TTA CCG AAC

-100 -90 -80 -70 -60
ACC GTG GGG TCG AGA TGA GTT TGC GTG CCG GGA CTC GGC AGA CTC GCC GCT CCG GCA

-50 -40 -30 -20 -10 1
CCG ACC GGG TGC ACC GGC ACC ACC CTC GAA ACG AGC GGA AGG ATG CAC ACA A

3. The process of claim 1 or 2 wherein said region the regulatory region is operatively linked to the LEP-10 export signal sequence located within said subfragment.

4. The process of claim 1, 2 or 3 wherein the regulatory region is operatively linked to a heterologous coding sequence.

5. A process for preparing a recombinant DNA molecule comprising the LEP-10 gene expression unit located within a 2.97kb BamHI - PstI fragment of a 4kb PstI fragment of S. lividans 1326 chromosomal DNA, which process comprises fusing appropriate DNA fragments.

6. A process for preparing an isolated DNA molecule having the sequence 
-390 -380 -370 -360 -350 -340
GCA CGG GGA TGT AGG GGA GTC GGG CGG AGG CCG CGA CCG AGG CCA GGG CGC GTC CCG

-330 -320 -310 -300 -290 -280
GCG TGC ACC GAC AGC AGG TTG TCC ATC CGC GCC GCC ACC AGC TCC CGG TCG CGG CCC

-270 -260 -250 -240 -230
AGG TAG CCG GGG GCT CCA CGG AGT GCG TCA TCA GGT CCC AGC CGG TGA ACC TCG CCG
 
-220 -210 -200 -190 -180 -170
GCC AGC CCC [?]CC TCG TCC TCC AGG AAC GCT AAT CAA GAA ACG CCC AGT TCC GGA CTT

-160 -150 -140 -130 -120 -110
GGA ACG TTC TAA TTC TGT GAC TTC ACG CCA CTG ATT CAA TAC GCA AGG TTA CCG AAC

-100 -90 -80 -70 -60
ACC GTG GGG TCG AGA TGA GTT TGC GTG CCG GGA CTC GGC AGA CTC GCC GCT CCG GCA

-50 -40 -30 -20 -10 1
CCG ACC GGG TGC ACC GGC ACC ACC CTC GAA ACG AGC GGA AGG ATG CAC ACA ATG CGG
Met Arg

10 20 30 40 50 60
AAC ACC GCG CGC TGG GCA GCG ACT CTG GGC CTG ACG GCC ACC GCC GTC TGC GGG CCC
Asn Thr Ala Arg Trp Ala Ala Thr Leu Gly Leu Thr Ala Thr Ala Val Cys Gly Pro

70 80 90 100 SIGNAL CLEAVAGE 110 120
CTC GCC GGG GCC TCC CTC GCC TCC CCG GCC ACC GCC CCC GCG TCG CTC TAC GCC CCC
Leu Ala Gly Ala Ser Leu Ala Ser Pro Ala Thr Ala Pro Ala Ser Leu Tyr Ala Pro

130 140 150 160 170
TCG GCC CTG GTG CTG ACC GTG GGG CAC GGA GAG AGC GCT GCC ACC GCC GCA CCC CTG
Ser Ala Leu Val Leu Thr Val Gly His Gly Glu Ser Ala Ala Thr Ala Ala Pro Leu

180 190 200 210 220 230
CGC GCG GTC ACC CTG ACC TGC GCC CCG ACC GCC TCC GGC ACC CAC CCG GCG GCC GCC
Arg Ala Val Thr Leu Thr Cys Ala Pro Thr Ala Ser Gly Thr His Pro Ala Ala Ala

240 250 260 270 280 290
GCG GCC TGT GCC GAA CTG CGC GCC GCG CAC GGC GAC CCG AGT GCC CTG GCC GCC GAG
Ala Ala Cys Ala Glu Leu Arg Ala Ala His Gly Asp Pro Ser Ala Leu Ala Ala Glu

300 310 320 330 340
GAC TCG GTG ATG TGC ACC CGG GAG TAC GCC CCC GTG GTC GTC ACC GTC GAC GGC GTC
Asp Ser Val Met Cys Thr Arg Glu Tyr Ala Pro Val Val Val Thr Val Asp Gly Val

350 360 370 380 390 400
TGG CAG GGG CGG CGC CTC TCC TAC GAA CGC ACC TTC GCC AAC GAG TGC GTG AAG AAC
Trp Gln Gly Arg Arg Leu Ser Tyr Glu Arg Thr Phe Ala Asn Glu Cys Val Lys Asn

410 420 430 440 450 460
GCG GGC AGC GCG AGC GTC TTC ACG TTC TGA GGG ACC GGG ACC GCC GGA CTG CGC GTG
Ala Gly Ser Ala Ser Val Phe Thr Phe ---

470 480 490 500 510
ATC GGC TGC TCG CTA CTG GGG AGT GCG AGC GCC GCC GTA CGG GTC CCG CGG GCC CGC

520 530 540 550 560 570
ACC GGG GAC GGC GGA CGG AGT GGG GCC GTC CGC CGT TCT CCC GGT TCA GGG GCA CGG

580 590 600 610 620 630
TCG GCC GTT CGC GGC CGG GCC GTC CGC GGC CCT GGC GGA CCG GGC ACG GTG GCT GTG

640 650
TCG CAC CAC GGA TAG CGC CGA CTG
 which includes the LEP-10 encoding region, which process comprises fusing appropriate DNA fragments.

7. A process for preparing a vector comprising the DNA molecule defined in any one of claims 1 to 6, which process comprises fusing appropriate DNA fragments.

8. A process for preparing a host microorganism or cell transformed with the DNA molecule defined in any of claims 1 to 6 or the vector of claim 7, which process comprises transforming the host with the DNA molecule or vector.

9. A process for preparing an isolated DNA molecule consisting essentially of the LEP-10 regulatory/export region HinfI - BglI subfragment of the 2.97kb BamHI - PstI fragment of a 4kb PstI fragment of S. lividans 1326 chromosomal DNA, which process comprises fusing appropriate DNA fragments.

10. A process for preparing a DNA molecule comprising the LEP-10 regulatory region as defined in claim 1 or 9 which process comprises hybridizing the chromosomal DNA to a probe based on the LEP-10 regulatory region to identify chromosomal DNA regions which comprise the LEP-10 regulatory region and then isolating the identified chromosomal DNA regions by restriction with a restriction endonuclease.

11. A process for preparing a DNA molecule comprising the LEP-10 regulatory region operatively linked to the LEP-10 export signal sequence as defined in claim 3 or 9 which process comprises hybridizing the chromosomal DNA to a probe based on the LEP-10 regulatory region and export signal sequence to identify chromosomal DNA regions which comprise the LEP-10 regulatory region and export signal sequence and then isolating the identified chromosomal DNA regions by restriction with a restriction endonuclease.

12. A process for preparing a recombinant DNA molecule as defined in claim 4 which process comprises restricting a recombinant DNA molecule having all or a portion of the LEP-10 gene expression unit at a point downstream of the LEP-10 regulatory region and ligating to said portion a coding sequence for a heterologous peptide.

13. A process for preparing a recombinant DNA molecule comprising the LEP-10 gene expression unit as defined in claim 5 or 6 which process comprises hybridizing chromosomal DNA to a probe based on the LEP-10 gene expression unit to identify chromosomal DNA regions which comprises the LEP10 gene expression unit and then isolating the identified chromosomal DNA regions by restriction with a restriction endonuclease.

14. A process for producing a polypeptide in Streptomyces which comprises transforming Streptomyces organisms with the recombinant DNA molecule defined in claim 4, 5 or 6 and then culturing the transformed Streptomyces such that the polypeptide is expressed.

## Patentansprüche (Patentansprüche für folgende(n) Vertragsstaat(en): BE, CH, DE, FR, GB, IT, LU, NL, SE, GR)

1. Rekombinantes DNA-Molekül, umfassend den LEP-10-Regulatorbereich der sich auf dem HinfI-BglI-Subfragment des 2,97 kb umfassenden BamHI-PstI-Fragments eines 4kb umfassenden PstI-Fragments der chromosomalen DNA von S.lividans 1326 befindet.

2. DNA-Molekül nach Anspruch 1, wobei der Bereich die folgende Sequenz umfaßt:

-130 -120 -110
ATT CAA TAC GCA AGG TTA CCG AAC

-100 -90 -80 -70 -60
ACC GTG GGG TCG AGA TGA GTT TGC GTG CCG GGA CTC GGC AGA CTC GCC GCT CCG GCA

-50 -40 -30 -20 -10 1
CCG ACC GGG TGC ACC GGC ACC ACC CTC GAA ACG AGC GGA AGG ATG CAC ACA A

3. DNA-Molekül nach Anspruch 1 oder 2, wobei der Regulatorbereich funktionell verknüpft ist mit der LEP-10-Exportsignalsequenz, die sich in dem Subfragment befindet.

4. DNA-Molekül nach Anspruch 1, 2 oder 3, wobei der Regulatorbereich funktionell verknüpft ist mit einer heterologen codierenden Sequenz.

5. Rekombinantes DNA-Molekül, umfassend die LEP-10-Genexpressionseinheit, die sich innerhalb eines 2,97 kb umfassenden BamHI-PstI-Fragments eines 4 kb umfassenden PstI-Fragments der chromosomalen DNA von S. lividans 1326 befindet.

6. Isoliertes DNA-Molekül mit der folgenden Sequenz 
-390 -380 -370 -360 -350 -340
GCA CGG GGA TGT AGG GGA GTC GGG CGG AGG CCG CGA CCG AGG CCA GGG CGC GTC CCG

-330 -320 -310 -300 -290 -280
GCG TGC ACC GAC AGC AGG TTG TCC ATC CGC GCC GCC ACC AGC TCC CGG TCG CGG CCC

-270 -260 -250 -240 -230
AGG TAG CCG GGG GCT CCA CGG AGT GCG TCA TCA GGT CCC AGC CGG TGA ACC TCG CCG

-220 -210 -200 -190 -180 -170
GCC AGC CCC [?]CC TCG TCC TCC AGG AAC GCT AAT CAA GAA ACG CCC AGT TCC GGA CTT

-160 -150 -140 -130 -120 -110
GGA ACG TTC TAA TTC TGT GAC TTC ACG CCA CTG ATT CAA TAC GCA AGG TTA CCG AAC

-100 -90 -80 -70 -60
ACC GTG GGG TCG AGA TGA GTT TGC GTG CCG GGA CTC GGC AGA CTC GCC GCT CCG GCA

-50 -40 -30 -20 -10 1
CCG ACC GGG TGC ACC GGC ACC ACC CTC GAA ACG AGC GGA AGG ATG CAC ACA ATG CGG
Met Arg

10 20 30 40 50 60
AAC ACC GCG CGC TGG GCA GCG ACT CTG GGC CTG ACG GCC ACC GCC GTC TGC GGG CCC
Asn Thr Ala Arg Trp Ala Ala Thr Leu Gly Leu Thr Ala Thr Ala Val Cys Gly Pro

70 80 90 100 SIGNAL CLEAVAGE 110 120
CTC GCC GGG GCC TCC CTC GCC TCC CCG GCC ACC GCC CCC GCG | TCG CTC TAC GCC CCC
Leu Ala Gly Ala Ser Leu Ala Ser Pro Ala Thr Ala Pro Ala | Ser Leu Tyr Ala Pro

130 140 150 160 170
TCG GCC CTG GTG CTG ACC GTG GGG CAC GGA GAG AGC GCT GCC ACC GCC GCA CCC CTC
Ser Ala Leu Val Leu Thr Val Gly His Gly Glu Ser Ala Ala Thr Ala Ala Pro Leu

180 190 200 210 220 230
CGC GCG GTC ACC CTG ACC TGC GCC CCG ACC GCC TCC GGC ACC CAC CCG GCG GCC GCC
Arg Ala Val Thr Leu Thr Cys Ala Pro Thr Ala Ser Gly Thr His Pro Ala Ala Ala

240 250 260 270 280 290
GCG GCC TGT GCC GAA CTG CGC GCC GCG CAC GGC GAC CCG AGT GCC CTG GCC GCC GAG
Ala Ala Cys Ala Glu Leu Arg Ala Ala His Gly Asp Pro Ser Ala Leu Ala Ala Glu

300 310 320 330 340
GAC TCG GTG ATG TGC ACC CGG GAG TAC GCC CCC GTG GTC GTC ACC GTC GAC GGC GTC
Asp Ser Val Met Cys Thr Arg Glu Tyr Ala Pro Val Val Val Thr Val Asp Gly Val

350 360 370 380 390 400
TGG CAG GGG CGG CGC CTC TCC TAC GAA CGC ACC TTC GCC AAC GAG TGC GTG AAG AAC
Trp Gln Gly Arg Arg Leu Ser Tyr Glu Arg Thr Phe Ala Asn Glu Cys Val Lys Asn

410 420 430 440 450 460
GCG GGC AGC GCG AGC GTC TTC ACG TTC TGA GGG ACC GGG ACC GCC GGA CTG CGC GTG
Ala Gly Ser Ala Ser Val Phe Thr Phe ---

470 480 490 500 510
ATC GGC TGC TCG CTA CTG GGG AGT GCG AGC GCC GCC GTA CGG GTC CCG CGG GCC CGC

520 530 540 550 560 570
ACC GCG GAC GCC GGA CGG AGT GGG GCC GTC CGC CGT TCT CCC GGT TCA CGG GCA CGG
 
580 590 600 610 620 630
TCG GCC GTT CGC GGC CGG GCC GTC CGC GGC CCT GGC GGA CCG GGC ACG GTG GCT GTG

640 650
TCG CAC CAC GGA TAG CGC CGA CTG
 die den LEP-10 codierenden Bereich umfaßt.

7. Vektor, umfassend das DNA-Molekül nach einem der Ansprüche 1 bis 6.

8. Wirtsorganismus oder -zelle, transformiert mit dem DNA-Molekül nach einem der Ansprüche 1 bis 6 oder dem Vektor nach Anspruch 7.

9. Isoliertes DNA-Molekül, im wesentlichen bestehend aus dem LEP-10-Regulator/Exportbereich des HinfI-BglI-Subfragments des 2,97 kb umfassenden BamHI-PstI-Fragments eines 4 kb umfassenden PstI-Fragments der chromosomalen DNA von S. lividans 1326.

10. Verfahren zur Herstellung eines DNA-Moleküls, umfassend den LEP-10-Regulatorbereich nach Anspruch 1 oder 9, umfassend die Hybridisierung der chromosomalen DNA an eine Sonde, die auf dem LEP-10-Regulatorbereich basiert, zur Identifizierung von chromosomalen DNA-Bereichen, die den LEP-10-Regulatorbereich umfassen, und danach Isolierung der identifizierten chromosomalen DNA-Bereiche durch Restriktion mit einer Restriktionsendonuclease.

11. Verfahren zur Herstellung eines DNA-Moleküls, umfassend den LEP-10-Regulatorbereich funktionell verknüpft mit der LEP-10-Exportsignalsequenz nach Anspruch 3 oder 9, umfassend die Hybridisierung der chromosomalen DNA an eine Sonde, die auf dem LEP-10-Regulatorbereich und der Exportsignalsequenz beruht, zur Identifizierung von chromosomalen DNA-Bereichen, die den LEP-10-Regulatorbereich und die Exportsignalsequenz umfassen, und danach Isolierung der identifizierten chromosomalen DNA-Bereiche durch Restriktion mit einer Restriktionsendonuclease.

12. Verfahren zur Herstellung eines rekombinanten DNA-Moleküls nach Anspruch 4, umfassend die Restriktion eines rekombinanten DNA-Molekül mit der gesamten oder einem Teil der LEP-10-Genexpressionseinheit an einem Punkt stromabwärts des LEP-10-Regulatorbereichs und Ligierung einer ein heterologes Peptid codierenden Sequenz an diesen Teil.

13. Verfahren zur Herstellung eines rekombinanten DNA-Moleküls, umfassend die LEP10-Genexpressionseinheit nach Anspruch 5 oder 6, umfassend die Hybridisierung von chromosomaler DNA an eine Sonde, die auf der LEP-10-Genexpressionseinheit basiert, zur Identifizierung von chromosomalen DNA-Bereichen, umfassend die LEP-10-Genexpressionseinheit, und danach Isolierung der identifizierten chromosomalen DNA-Bereiche durch Restriktion mit einer Restriktionsendonuclease.

14. Verfahren zur Herstellung eines Polypeptids in Streptomyces, umfassend die Transformation von Streptomyces-Organismen mit dem rekombinanten DNA-Molekül nach Anspruch 4, 5 oder 6 und danach Züchtung des transformierten Streptomyces, so daß das Polypeptid exprimiert wird.

15. Verfahren zur Herstellung eines DNA-Moleküls gemäß der Definition in einem der Ansprüche 1 bis 6 oder 9 oder eines Vektors gemäß der Definition in Anspruch 7, umfassend die Fusion geeigneter DNA-Fragmente.

## Patentansprüche (Patentansprüche für folgende(n) Vertragsstaat(en): AT, ES)

1. Verfahren zur Herstellung eines rekombinanten DNA-Moleküls, umfassend den LEP-10-Regulatorbereich der sich auf dem HinfI-BglI-Subfragment des 2,97 kb umfassenden BamHI-PstI-Fragments eines 4kb umfassenden PstI-Fragments der chromosomalen DNA von S.lividans 1326 befindet, umfassend die Fusion geeigneter DNA-Fragmente.

2. Verfahren nach Anspruch 1, wobei der Bereich die folgende Sequenz umfaßt:

-130 -120 -110
ATT CAA TAC GCA AGG TTA CCG AAC

-100 -90 -80 -70 -60
ACC GTC GGG TCG AGA TGA GTT TGC GTG CCG GGA CTC GGC AGA CTC GCC GCT CCG GCA

-50 -40 -30 -20 -10 1
CCG ACC GGG TGC ACC GGC ACC ACC CTC GAA ACG AGC GGA AGG ATG CAC ACA A

3. Verfahren nach Anspruch 1 oder 2, wobei der Regulatorbereich funktionell verknüpft ist mit der LEP-10-Exportsignalsequenz, die sich in dem Subfragment befindet.

4. Verfahren nach Anspruch 1, 2 oder 3, wobei der Regulatorbereich funktionell verknüpft ist mit einer heterologen codierenden Sequenz.

5. Verfahren zur Herstellung eines rekombinanten DNA-Moleküls, umfassend die LEP-10-Genexpressionseinheit, die sich innerhalb eines 2,97 kb umfassenden BamHI-PstI-Fragments eines 4 kb umfassenden PstI-Fragments der chromosomalen DNA von S. lividans 1326 befindet, umfassend die Fusion geeigneter DNA-Fragmente.

6. Verfahren zur Herstellung eines isolierten DNA-Moleküls mit der folgenden Sequenz 
-390 -380 -370 -360 -350 -340
GCA CGG GGA TGT AGG GGA GTC GGG CGG AGG CCG CGA CCG AGG CCA GGG CGC GTC CCG

-330 -320 -310 -300 -290 -280
GCG TGC ACC GAC AGC AGG TTG TCC ATC CGC GCC GCC ACC AGC TCC CGG TCG CGG CCC

-270 -260 -250 -240 -230
AGG TAG CCG GGG GCT CCA CGG AGT GCG TCA TCA GGT CCC AGC CGG TGA ACC TCG CCG

-220 -210 -200 -190 -180 -170
GCC AGC CCC [?]CC TCG TCC TCC AGG AAC GCT AAT CAA GAA ACG CCC AGT TCC GGA CTT

-160 -150 -140 -130 -120 -110
GGA ACG TTC TAA TTC TGT GAC TTC ACG CCA CTG ATT CAA TAC GCA AGG TTA CCG AAC

-100 -90 -80 -70 -60
ACC GTG GGG TCG AGA TGA GTT TGC GTG CCG GGA CTC GGC AGA CTC GCC GCT CCG GCA

-50 -40 -30 -20 -10 1
CCG ACC GGG TGC ACC GGC ACC ACC CTC GAA ACG AGC GGA AGG ATG CAC ACA ATG CGC
Met Arg

10 20 30 40 50 60
AAC ACC GCG CGC TGG GCA GCG ACT CTG GGC CTG ACG GCC ACC GCC GTC TGC GGG CCC
Asn Thr Ala Arg Trp Ala Ala Thr Leu Gly Leu Thr Ala Thr Ala Val Cys Gly Pro

70 80 90 100 SIGNAL CLEAVAGE 110 120
CTC GCC GGG GCC TCC CTC GCC TCC CCG GCC ACC GCC CCC GCG TCG CTC TAC GCC CCC
Leu Ala Gly Ala Ser Leu Ala Ser Pro Ala Thr Ala Pro Ala Ser Leu Tyr Ala Pro

130 140 150 160 170
TCG GCC CTG GTG CTG ACC GTG GGG CAC GGA GAG AGC GCT GCC ACC GCC GCA CCC CTG
Ser Ala Leu Val Leu Thr Val Gly His Gly Glu Ser Ala Ala Thr Ala Ala Pro Leu

180 190 200 210 220 230
CGC GCG GTC ACC CTG ACC TGC GCC CCG ACC GCC TCC GGC ACC CAC CCG GCG GCC GCC
Arg Ala Val Thr Leu Thr Cys Ala Pro Thr Ala Ser Gly Thr His Pro Ala Ala Ala

240 250 260 270 280 290
GCG GCC TGT GCC GAA CTG CGC GCC GCG CAC GGC GAC CCG AGT GCC CTG GCC GCC GAG
Ala Ala Cys Ala Glu Leu Arg Ala Ala His Gly Asp Pro Ser Ala Leu Ala Ala Glu

300 310 320 330 340
GAC TCG GTG ATG TGC ACC CGG GAG TAC GCC CCC GTG GTC GTC ACC GTC GAC GGC GTC
Asp Ser Val Met Cys Thr Arg Glu Tyr Ala Pro Val Val Val Thr Val Asp Gly Val

350 360 370 380 390 400
TGG CAG GGG CGG CGC CTC TCC TAC GAA CGC ACC TTC GCC AAC GAG TGC GTG AAG AAC
Trp Gln Gly Arg Arg Leu Ser Tyr Glu Arg Thr Phe Ala Asn Glu Cys Val Lys Asn

410 420 430 440 450 460
GCG GGC AGC GCG AGC GTC TTC ACG TTC TGA GGG ACC GGG ACC GCC GGA CTG CGC GTG
Ala Gly Ser Ala Ser Val Phe Thr Phe ---

470 480 490 500 510
ATC GGC TGC TCG CTA CTG GGG AGT GCG AGC GCC GCC GTA CGG GTC CCG CGG GCC CGC
 
520 530 540 550 560 570
ACC GGG GAC GGC GGA CGG AGT GGG GCC GTC CGC CGT TCT CCC GGT TCA GGG GCA CGG

580 590 600 610 620 630
TCG GCC GTT CGC GGC CGG GCC GTC CGC GGC CCT GGC GGA CCG GGC ACG GTG GCT GTG

640 650
 die den LEP-10 codierenden Bereich umfaßt, umfassend die Fusion geeigneter DNA-Fragmente.

7. Verfahren zur Herstellung eines Vektors, der das DNA-Molekül nach einem der Ansprüche 1 bis 6 umfaßt, umfassend die Fusion geeigneter DNA-Fragmente.

8. Verfahren zur Herstellung eines Wirtsorganismus oder -zelle, transformiert mit dem DNA-Molekül nach einem der Ansprüche 1 bis 6 oder dem Vektor nach Anspruch 7, umfassend die Transformation des Wirtes mit dem DNA-Molekül oder dem Vektor.

9. Verfahren zur Herstellung eines isolierten DNA-Moleküls, im wesentlichen bestehend aus dem LEP-10-Regulator/Exportbereich des HinfI-BglI-Subfragments des 2,97 kb umfassenden BamHI-PstI-Fragments eines 4 kb umfassenden PstI-Fragments der chromosomalen DNA von S. lividans 1326, umfassend die Fusion geeigneter DNA-Fragmente.

10. Verfahren zur Herstellung eines DNA-Moleküls, umfassend den LEP-10-Regulatorbereich nach Anspruch 1 oder 9, umfassend die Hybridisierung der chromosomalen DNA an eine Sonde, die auf dem LEP-10-Regulatorbereich basiert, zur Identifizierung von chromosomalen DNA-Bereichen, die den LEP-10-Regulatorbereich umfassen, und danach Isolierung der identifizierten chromosomalen DNA-Bereiche durch Restriktion mit einer Restriktionsendonuclease.

11. Verfahren zur Herstellung eines DNA-Moleküls, umfassend den LEP-10-Regulatorbereich funktionell verknüpft mit der LEP-10-Exportsignalsequenz nach Anspruch 3 oder 9, umfassend die Hybridisierung der chromosomalen DNA an eine Sonde, die auf dem LEP-10-Regulatorbereich und der Exportsignalsequenz beruht, zur Identifizierung von chromosomalen DNA-Bereichen, die den LEP-10-Regulatorbereich und die Exportsignalsequenz umfassen, und danach Isolierung der identifizierten chromosomalen DNA-Bereiche durch Restriktion mit einer Restriktionsendonuclease.

12. Verfahren zur Herstellung eines rekombinanten DNA-Moleküls nach Anspruch 4, umfassend die Restriktion eines rekombinanten DNA-Molekül mit der gesamten oder einem Teil der LEP-10-Genexpressionseinheit an einem Punkt stromabwärts des LEP-10-Regulatorbereichs und Ligierung einer ein heterologes Peptid codierenden Sequenz an diesen Teil.

13. Verfahren zur Herstellung eines rekombinanten DNA-Moleküls, umfassend die LEP10-Genexpressionseinheit nach Anspruch 5 oder 6, umfassend die Hybridisierung von chromosomaler DNA an eine Sonde, die auf der LEP-10-Genexpressionseinheit basiert, zur Identifizierung von chromosomalen DNA-Bereichen, umfassend die LEP-10-Genexpressionseinheit, und danach Isolierung der identifizierten chromosomalen DNA-Bereiche durch Restriktion mit einer Restriktionsendonuclease.

14. Verfahren zur Herstellung eines Polypeptids in Streptomyces, umfassend die Transformation von Streptomyces-Organismen mit dem rekombinanten DNA-Molekül nach Anspruch 4, 5 oder 6 und danach Züchtung des transformierten Streptomyces, so daß das Polypeptid exprimiert wird.

## Revendications (Revendications pour l'(les) Etat(s) contractant(s) suivant(s): BE, CH, DE, FR, GB, IT, LU, NL, SE, GR)

1. Molécule d'ADN recombinant comprenant la région régulatrice LEP-10 située sur le sous-fragment HinfI-BglI du fragment BamHI-PstI de 2,97 kb, du fragment PstI de 4kb de l'ADN chromosomal de S. lividans 1326.

2. Molécule d'ADN suivant la revendication 1, dans laquelle ladite région comprend la séquence :

-130 -120 -110
ATT CAA TAC GCA AGG TTA CCG AAC

-100 -90 -80 -70 -60
ACC GTG GGG TCG AGA TGA GTT TGC GTG CCG GGA CTC GGC AGA CTC GCC GCT CCG GCA

-50 -40 -30 -20 -10 1
CCG ACC GGG TGC ACC GGC ACC ACC CTC GAA ACG AGC GGA AGG ATG CAC ACA A

3. Molécule d'ADN suivant la revendication 1 ou 2, dans laquelle la région régulatrice est liée de façon opérationnelle à la séquence de signal export LEP-10.

4. Molécule d'ADN suivant la revendication 1, 2, ou 3, dans laquelle la région régulatrice est liée de façon opérationnelle à une séquence codante hétérologue.

5. Molécule d'ADN recombinant comprenant l'unité d'expression du gène LEP-10 dans un fragment BamHI-PstI de 2.97 kb, du fragment PstI de 4kb de l'ADN chromosomal de S. lividans 1326.

6. Molécule d'ADN isolée ayant la séquence : 
-390        -380        -370        -360        -350        -340
•           •           •           •           •           •
GCA CGG GGA TGT AGG GGA GTC GGG CGG AGG CCG CGA CCG AGG CCA GGG CGC GTC CCG

-330        -320        -310        -300        -290        -280
•           •           •           •           •           •
GCG TGC ACC GAC AGC AGG TTG TCC ATC CGC GCC GCC ACC AGC TCC CGG TCG CGG CCC

-270        -260        -250        -240        -230
•           •           •           •           •
AGG TAG CCG GGG GCT CCA CGG AGT GCG TCA TCA GGT CCC AGC CGG TGA ACC TCG CCG

-220        -210        -200        -190        -180        -170
•           •           •           •           •           •
GCC AGC CCC [?]CC TCG TCC TCC AGG AAC GCT AAT CAA GAA ACG CCC AGT TCC GGA CTT

-160        -150        -140        -130        -120        -110
•           •           •           •           •           •
GGA ACG TTC TAA TTC TGT GAC TTC ACG CCA CTG ATT CAA TAC GCA AGG TTA CCG AAC

-100        -90         -80         -70         -60
•           •           •           •           •
ACC GTG GGG TCG AGA TGA GTT TGC GTG CCG GGA CTC GGC AGA CTC GCC GCT CCG GCA
 
-50 -40 -30 -20 -10 1
CCG ACC GGG TGC ACC GGC ACC ACC CTC GAA ACG AGC GGA AGG ATG CAC ACA ATG CGG
Met Arg

10 20 30 40 50 60
AAC ACC GCG CGC TGG GCA GCG ACT CTG GGC CTG ACG GCC ACC GCC GTC TGC GGG CCC
Asn Thr Ala Arg Trp Ala Ala Thr Leu Gly Leu Thr Ala Thr Ala Val Cys Gly Pro

70 80 90 100 SIGNAL CLEAVAGE 110 120
CTC GCC GGG GCC TCC CTC GCC TCC CCG GCC ACC GCC CCC GCG | TCG CTC TAC GCC CCC
Leu Ala Gly Ala Ser Leu Ala Ser Pro Ala Thr Ala Pro Ala | Ser Leu Tyr Ala Pro

130 140 150 160 170
TCG GCC CTG GTG CTG ACC GTG GGG CAC GGA GAG AGC GCT GCC ACC GCC GCA CCC CTG
Ser Ala Leu Val Leu Thr Val Gly His Gly Glu Ser Ala Ala Thr Ala Ala Pro Leu

180 190 200 210 220 230
CGC GCG GTC ACC CTG ACC TGC GCC CCG ACC GCC TCC GGC ACC CAC CCG GCG GCC GCC
Arg Ala Val Thr Leu Thr Cys Ala Pro Thr Ala Ser Gly Thr His Pro Ala Ala Ala

240 250 260 270 280 290
GCG GCC TGT GCC GAA CTG CGC GCC GCG CAC GGC GAC CCG AGT GCC CTG GCC GCC GAG
Ala Ala Cys Ala Glu Leu Arg Ala Ala His Gly Asp Pro Ser Ala Leu Ala Ala Glu

300 310 320 330 340
GAC TCG GTG ATG TGC ACC CGG GAG TAC GCC CCC GTG GTC GTC ACC GTC GAC GGC GTC
Asp Ser Val Met Cys Thr Arg Glu Tyr Ala Pro Val Val Val Thr Val Asp Gly Val

350 360 370 380 390 400
TGG CAG GGG CGG CGC CTC TCC TAC GAA CGC ACC TTC GCC AAC GAG TGC GTG AAG AAC
Trp Gln Gly Arg Arg Leu Ser Tyr Glu Arg Thr Phe Ala Asn Glu Cys Val Lys Asn

410 420 430 440 450 460
GCG GGC AGC GCG AGC GTC TTC ACG TTC TGA GGG ACC GGG ACC GCC GGA CTG CGC GTG
Ala Gly Ser Ala Ser Val Phe Thr Phe ---

470 480 490 500 510
ATC GGC TGC TCG CTA CTG GGG AGT GCG AGC GCC GCC GTA CGG GTC CCG CGG GCC CGC

520 530 540 550 560 570
ACC GGG GAC GGC GGA CGG AGT GGG GCC GTC CGC CGT TCT CCC GGT TCA GGG GCA CGG

580 590 600 610 620 630
TCG GCC GTT CGC GGC CGG GCC GTC CGC GGC CCT GGC GGA CCG GGC ACG GTG GCT GTG

640 650
TCG CAC CAC GGA TAG CGC CGA CTG
 qui inclut la région encodante LEP-10.

7. Vecteur comprenant la molécule d'ADN suivant l'une quelconque des revendications 1 à 6.

8. Micro-organisme ou cellule-hôte transformé par la molécule d'ADN suivant l'une quelconque des revendications 1 à 6, ou le vecteur suivant la revendication 7.

9. Molécule d'ADN isolée consistant essentiellement en un sous-fragment HinfI-BglI de la région régulatrice/export LEP-10 du fragment BamHI-PstI de 2,97 kb, d'un fragment PstI de 4kb de l'ADN chromosomal de S. lividans 1326.

10. Procédé de préparation d'une molécule d'ADN comprenant la région régulatrice LEP-10 suivant la revendication 1, ou 9, lequel procédé comprend l'hybridation de l'ADN chromosomal à une sonde basée sur la région régulatrice LEP-10, pour identifier les régions d'ADN chromosomal qui comprennent le région régulatrice LEP-10, et ensuite l'isolement des régions identifiées d'ADN chromosomal, par restriction au moyen d'une endonucléase de restriction.

11. Procédé de préparation d'une molécule d'ADN comprenant la région régulatrice liée de façon opérationnelle à la séquence de signal export LEP-10 suivant la revendication 3 ou 9, lequel procédé comprend l'hybridation de l'ADN chromosomal à une sonde basée sur la région régulatrice et le signal export LEP-10, pour identifier les régions d'ADN chromosomal qui comprennent le région régulatrice LEP-10, et ensuite l'isolement des régions identifiées d'ADN chromosomal, par restriction au moyen d'une endonucléase de restriction.

12. Procédé de préparation d'une molécule d'ADN recombinant suivant la revendication 4, lequel procédé comprend la restriction d'une molécule d'ADN ayant complètement ou en partie, l'unité d'expression du gène LEP-10 en un point en aval de la région régulatrice, et la ligation à ladite portion, d'une séquence codant pour un peptide hétérologue.

13. Procédé de préparation d'une molécule d'ADN recombinant comprenant l'unité d'expression du gène LEP-10 suivant la revendication 5 ou 6, lequel procédé comprend l'hybridation de l'ADN chromosomal à une sonde basée sur l'unité d'expression du gène LEP-10 pour identifier les régions d'ADN chromosomal qui comprennent l'unité d'expression du gène LEP-10, et ensuite l'isolement des régions identifiées d'ADN chromosomal, par restriction au moyen d'une endonucléase de restriction.

14. Procédé de préparation d'un polypeptide dans un Streptomyces, qui comprend la transformation des organismes de Streptomyces avec la molécule d'ADN recombinant suivant la revendication 4, 5 ou 6, et ensuite la culture du Streptomyces transformé de façon à ce que le polypeptide soit exprimé.

15. Procédé de préparation d'une molécule d'ADN suivant l'une quelconque des revendications 1 à 6, ou 9, ou d'un vecteur suivant la revendication 7, lequel procédé comprend la fusion de segments appropriés d'ADN.

## Revendications (Revendications pour l'(les) Etat(s) contractant(s) suivant(s): AT, ES)

1. Procédé de préparation d'une molécule d'ADN recombinant comprenant la région régulatrice LEP-10 située sur le sousfragment HinfI-BglI du fragment BamHI-PstI de 2,97 kb, du fragment PstI de 4kb de l'ADN chromosomal de S. lividans 1326, lequel procédé comprend la fusion de fragments appropriés d'ADN.

2. Procédé suivant la revendication 1, dans lequel ladite région comprend la séquence :

-130 -120 -110
ATT CAA TAC GCA AGG TTA CCG AAC

-100 -90 -80 -70 -60
ACC GTG GGG TCG AGA TGA GTT TGC GTG CCG GGA CTC GGC AGA CTC GCC GCT CCG GCA

-50 -40 -30 -20 -10 1
CCG ACC GGG TGC ACC GGC ACC ACC CTC GAA ACG AGC GGA AGG ATG CAC ACA A

3. Procédé suivant la revendication 1, ou 2, dans lequel ladite région régulatrice est liée de façon opérationnelle à la séquence signal export LEP-10 située dans ledit sous-fragment.

4. Procédé suivant la revendication 1, 2, ou 3, dans lequel la région régulatrice est liée de façon opérationnelle à une séquence codante hétérologue.

5. Procédé de préparation d'une molécule d'ADN recombinant comprenant l'unité d'expression du gène LEP-10 située dans un fragment BamHI-PstI de 2,97 kb, du fragment PstI de 4kb de l'ADN chromosomal de S. lividans 1326, lequel procédé comprend la fusion de fragments appropriés d'ADN.

6. Procédé de préparation d'une molécule d'ADN isolée ayant la séquence : 
-390 -380 -370 -360 -350 -340
GCA CGG GGA TGT AGG GGA GTC GGG CGG AGG CCG CGA CCG AGG CCA GGG CGC GTC CCG

-330 -320 -310 -300 -290 -280
GCG TGC ACC GAC AGC AGG TTG TCC ATC CGC GCC GCC ACC AGC TCC CGG TCG CGG CCC

-270 -260 -250 -240 -230
AGG TAG CCG GGG GCT CCA CGG AGT GCG TCA TCA GGT CCC AGC CGG TGA ACC TCG CCG
 
-220 -210 -200 -190 -180 -170
GCC AGC CCC [?]CC TCG TCC TCC AGG AAC GCT AAT CAA GAA ACG CCC AGT TCC GGA CTT

-160 -150 -140 -130 -120 -110
GGA ACG TTC TAA TTC TGT GAC TTC ACG CCA CTG ATT CAA TAC GCA AGG TTA CCG AAC

-100 -90 -80 -70 -60
ACC GTG GGG TCG AGA TGA GTT TGC GTG CCG GGA CTC GGC AGA CTC GCC GCT CCG GCA

-50 -40 -30 -20 -10 1
CCG ACC GGG TGC ACC GGC ACC ACC CTC GAA ACG AGC GGA AGG ATG CAC ACA ATG CGG
Met Arg

10 20 30 40 50 60
AAC ACC GCG CGC TGG GCA GCG ACT CTG GGC CTG ACG GCC ACC GCC GTC TGC GGG CCC
Asn Thr Ala Arg Trp Ala Ala Thr Leu Gly Leu Thr Ala Thr Ala Val Cys Gly Pro

70 80 90 100 SIGNAL CLEAVAGE 110 120
CTC GCC GGG GCC TCC CTC GCC TCC CCG GCC ACC GCC CCC GCG|TCG CTC TAC GCC CCC
Leu Ala Gly Ala Ser Leu Ala Ser Pro Ala Thr Ala Pro Ala|Ser Leu Tyr Ala Pro

130 140 150 160 170
TCG GCC CTG GTG CTG ACC GTG GGG CAC GGA GAG AGC GCT GCC ACC GCC GCA CCC CTG
Ser Ala Leu Val Leu Thr Val Gly His Gly Glu Ser Ala Ala Thr Ala Ala Pro Leu

180 190 200 210 220 230
CGC GCG GTC ACC CTG ACC TGC GCC CCG ACC GCC TCC GGC ACC CAC CCG GCG GCC GCC
Arg Ala Val Thr Leu Thr Cys Ala Pro Thr Ala Ser Gly Thr His Pro Ala Ala Ala

240 250 260 270 280 290
GCG GCC TGT GCC GAA CTG CGC GCC GCG CAC GGC GAC CCG AGT GCC CTG GCC GCC GAG
Ala Ala Cys Ala Glu Leu Arg Ala Ala His Gly Asp Pro Ser Ala Leu Ala Ala Glu

300 310 320 330 340
GAC TCG GTG ATG TGC ACC CGG GAG TAC GCC CCC GTG GTC GTC ACC GTC GAC GGC GTC
Asp Ser Val Met Cys Thr Arg Glu Tyr Ala Pro Val Val Val Thr Val Asp Gly Val

350 360 370 380 390 400
TGG CAG GGG CGG CGC CTC TCC TAC GAA CGC ACC TTC GCC AAC GAG TGC GTG AAG AAC
Trp Gln Gly Arg Arg Leu Ser Tyr Glu Arg Thr Phe Ala Asn Glu Cys Val Lys Asn

410 420 430 440 450 460
GCG GGC AGC GCG AGC GTC TTC ACG TTC TGA GGG ACC GGG ACC GCC GGA CTG CGC GTG
Ala Gly Ser Ala Ser Val Phe Thr Phe ---

470 480 490 500 510
ATC GGC TGC TCG CTA CTG GGG AGT GCG AGC GCC GCC GTA CGG GTC CCG CGG GCC CGC

520 530 540 550 560 570
ACC GGG GAC GGC GGA CGG AGT GGG GCC GTC CGC CGT TCT CCC GGT TCA GGG GCA CGG

580 590 600 610 620 630
TCG GCC GTT CGC GGC CGG GCC GTC CGC GGC CCT GGC GGA CCG GGC ACG GTG GCT GTG

640 650
TCG CAC CAC GGA TAG CGC CGA CTG
 qui inclut la région encodante LEP-10, lequel procédé comprend la fusion de fragments appropriés d'ADN.

7. Procédé de préparation d'un vecteur comprenant la molécule d'ADN suivant l'une quelconque des revendications 1 à 6, lequel procédé comprend la fusion de fragments appropriés d'ADN.

8. Procédé de préparation d'un micro-organisme ou cellulehôte transformé par la molécule d'ADN suivant l'une quelconque des revendications 1 à 6, ou le vecteur suivant la revendication 7, lequel procédé comprend la fusion de fragments appropriés d'ADN.

9. Procédé de préparation d'une molécule d'ADN isolée consistant essentiellement en un sous-fragment HinfI-BglI de la région régulatrice/export LEP-10 du fragment BamHI-PstI de 2,97 kb, d'un fragment PstI de 4kb de l'ADN chromosomal de S. lividans 1326, lequel procédé comprend la fusion de fragments appropriés d'ADN.

10. Procédé de préparation d'une molécule d'ADN comprenant la région régulatrice LEP-10 suivant la revendication 1, ou 9, lequel procédé comprend l'hybridation de l'ADN chromosomal à une sonde basée sur la région régulatrice LEP-10, pour identifier les régions d'ADN chromosomal qui comprennent le région régulatrice LEP-10, et ensuite l'isolement des régions identifiées d'ADN chromosomal, par restriction au moyen d'une endonucléase de restriction.

11. Procédé de préparation d'une molécule d'ADN comprenant la région régulatrice liée de façon opérationnelle à la séquence de signal export LEP-10 suivant la revendication 3 ou 9, lequel procédé comprend l'hybridation de l'ADN chromosomal à une sonde basée sur la région régulatrice et le signal export LEP-10, pour identifier les régions d'ADN chromosomal qui comprennent le région régulatrice LEP-10, et ensuite l'isolement des régions identifiées d'ADN chromosomal, par restriction au moyen d'une endonucléase de restriction.

12. Procédé de préparation d'une molécule d'ADN recombinant suivant la revendication 4, lequel procédé comprend la restriction d'une molécule d'ADN ayant complètement ou en partie, l'unité d'expression du gène LEP-10 en un point en aval de la région régulatrice, et la ligation à ladite portion, d'une séquence codant pour un peptide hétérologue.

13. Procédé de préparation d'une molécule d'ADN recombinant comprenant l'unité d'expression du gène LEP-10 suivant la revendication 5 ou 6, lequel procédé comprend l'hybridation de l'ADN chromosomal à une sonde basée sur l'unité d'expression du gène LEP-10 pour identifier les régions d'ADN chromosomal qui comprennent l'unité d'expression du gène LEP-10, et ensuite l'isolement des régions identifiées d'ADN chromosomal, par restriction au moyen d'une endonucléase de restriction.

14. Procédé de préparation d'un polypeptide dans un Streptomyces qui comprend la transformation des organismes de Streptomyces avec la molécule d'ADN recombinant suivant la revendication 4, 5 ou 6, et ensuite la culture du Streptomyces transformé de façon à ce que le polypeptide soit exprimé.
